(19) Europäisches Patentamt

European Patent Office

Office européen des brevets

(11) **EP 1 714 635 A1**

(12) **EUROPEAN PATENT APPLICATION**
published in accordance with Art. 158(3) EPC

(43) Date of publication:
25.10.2006 Bulletin 2006/43

(21) Application number: 05709428.6

(22) Date of filing: 28.01.2005

(51) Int Cl.:
*A61K 8/18* (2006.01)       *A61K 36/18* (2006.01)
*A23G 3/00* (2006.01)       *A23L 1/30* (2006.01)
*A61P 37/08* (2006.01)

(86) International application number:
**PCT/JP2005/001201**

(87) International publication number:
**WO 2005/072684 (11.08.2005 Gazette 2005/32)**

(84) Designated Contracting States:
**AT BE BG CH CY CZ DE DK EE ES FI FR GB GR HU IE IS IT LI LT LU MC NL PL PT RO SE SI SK TR**

(30) Priority: 28.01.2004 JP 2004019283

(71) Applicant: SUNTORY LIMITED
Osaka-shi,
Osaka 530-8203 (JP)

(72) Inventors:
• KATO, Megumi
6650015 (JP)

• SUWA, Yoshihide
5670009 (JP)
• FUKUI, Yuko
5691123 (JP)
• SASAYAMA, Aya
4210600 (JP)
• MATSUMOTO, Takehiro
5630032 (JP)

(74) Representative: Vossius & Partner
Siebertstrasse 4
81675 München (DE)

(54) **PROCESS FOR PRODUCING MACA EXTRACT**

(57) The present invention provides an efficient extraction method of Maca extract having safe anti-allergic effects enabling long-term intake, and a food and beverage product, a perfumery and a cosmetic as well as a pharmaceutical preparation comprising thus obtained Maca extract showing anti-allergic effect.

In the provided extraction method of Maca extract, the temperature of extracting solution is from 20°C to 75°C when Maca extract is extracted by adding aqueous solution containing ethanol being ethanol concentration of from 20 to 100 by volume%.

EP 1 714 635 A1

**Description**

TECHNICAL FIELD

The present invention relates to an extraction method of Maca extract from **Cruciferae** and to a food and a beverage product, a perfumery and a cosmetic, as well as a pharmaceutical preparation which comprising the Maca extract and having an anti-allergic activity.

TECHNICAL BACKGROUND

Up to the present, there have been many kinds of compounds having various pharmacological activities synthesized. Also, there have been found many pharmaceutical preparations effectively applied to various diseases, and clinically used. In that, chemosynthetic pharmaceutical preparations such as tranilast and sodium cromoglycate (herein after referred to as "DSCG") have been found as an anti-allergic agent. Additionally, a plant extract, such as *Tanacetum vulgara* extract containing large amount of caffeic acid, is used as 5-lipoxygenase inhibitors, which produce leukotriene from arachidonic acid.

However, an anti-allergic agent generally causes side effects such as drowsiness, dry throat and gastrointestinal distress, and thus long-term administration of the agent jeopardizes safety. On the other hand, a lot of food materials believed to contribute to treat allergic disease are searched. One example is the sweet tea **(leaves of *Rubes suavissimus*)** extract. Because the sweet tea is known to possess anti-allergic activity (Japanese Patent Laid-Open Publication No. Hei06-192114), the extract is comprised in, for example, throat lozenge and used to relieve a symptom of hay fever.

*Lepidium meyenii Walp* (herein after referred to as "Maca") is a *Cruciferae* originated in Andean Cordilleras of Peru in South America. Maca grows creeping on the earth and outspreading leaves, and its root shapes like a turnip. Maca is grown over 2,000 years in the Andes and taken for maintenance of good health. Main components of Maca are polysaccharides, proteins and so on. Moreover, main amino acids, especially, essential amino acids, which cannot be synthesized in body and therefore must be taken from food, are contained. Maca is also rich in various vitamins such as vitamin B, c, and E, and minerals such as calcium, iron and zinc. Accordingly, there are dozens of food items using Maca in Peru. Maca is familiar as health food such as cookies and juice comprising Maca named "Chicha de Maca", Maca liquor and eating Maca powder with Yogurt.

A positive effect of Maca is traditionally believed to be revitalizing effect, but recently compounds of Maca having anticancer effects and recovering reproductive function effects have been opened (American Patent Gazette No. 6267995 B1). There also have been suggested that using Maca with an antler would possess an effect to increase the testosterone concentration in a human (Japanese Translated National Publication of Patent Application No. 2003-523945). There are also reports which leads to the expectation for potentiality of Maca to show anti-inflammatory effect and to be anti-allergic agent (Igaku & Seibutsugaku: Vol.145, No.6, p81-86, 2002.12.10).

Many food and beverage products containing small pieces or powder of Maca or Maca extract are produced due to recent attentions to Maca's positive effects. Nevertheless, there hardly is any study on the method to extract the Maca extract efficiently, especially paying special attention to strong anti-allergic effect thereof, using solvent having high safety margin.

DISCLOSURE OF THE INVENTION

Accordingly, the objective of the present invention is to provide an efficient extraction method of Maca extract having safe anti-allergic effect enabling long-term intake, and to provide a food and beverage product, a perfumery and a cosmetic and a pharmaceutical preparation comprising thus obtained Maca extract to show anti-allergic effect.

The inventors of the present invention had studied and researched thoroughly to solve above objective, and surprisingly found that the potency of anti-allergic effect of the obtained extract differs greatly depending on the extraction condition. Especially, they have newly found that the extract having high potency of anti-allergic effect can be obtained if ethanol concentration and temperature of extracting solvent is maintained in specific range when aqueous solution containing ethanol is used as extracting solvent, and thus completed the invention.

MEANS TO SOLVE THE PROBLEMS

Accordingly, the present invention provides:

(1) an extraction method of Maca extract, wherein the temperature of extracting solution is from 20°C to 75°C when Maca extract is extracted by adding aqueous solution containing ethanol to small pieces or powder of Maca;
(2) the extraction method according to (1), wherein concentration of the ethanol in the aqueous solution containing ethanol is from 20 to 100 by volume%;

(3) the extraction method according to (1), wherein from 0.3 to 500 by weight% of the aqueous solution containing ethanol is used against 1 by weight% of small pieces or powder of Maca; and

(4) the extraction method according to any one of (1) to (3), wherein the small pieces or powder of Maca is a residue of watery extraction.

Additionally, the present invention provides:

(5) a Maca extract obtained by the method according to any one of

(1) to (4);

(6) a food and beverage product, a perfumery and a cosmetic and a pharmaceutical preparation comprising the Maca extract of (5);

(7) a food and beverage product, a perfumery and a cosmetic and a pharmaceutical preparation according to (6), wherein 0.01 to 99.5% of Maca extract according to (5) in dry weight is comprised;

(8) a food and beverage product, a perfumery and a cosmetic and a pharmaceutical preparation according to (6) or (7), possessing an anti-allergic effect;

(9) an additive or a compounding agent for a food and beverage product, a perfumery and a cosmetic, or a pharmaceutical preparation, wherein Maca extract according to (5) is comprised; and

(10) a food and beverage product, a perfumery and a cosmetic or a pharmaceutical preparation according to (8), being a candy, a troche, a gum, a granule or a tablet.

EFFECT OF THE INVENTION

The present invention provides the Maca extract having a high potency of anti-allergic effect and suitable for oral administration. The present invention further provides a food and a beverage product, a perfumery and a cosmetic, and a pharmaceutical preparation having anti-allergic effects, by comprising the Maca extract thereof or its dry product. Yet further, the present invention provides an additives and compounding agent for use in the production of a food and a beverage product, a perfumery and a cosmetic, and a pharmaceutical preparation thereof.

BEST MODE FOR CARRYING OUT THE INVENTION

The main raw material to obtain the extract by the present invention is *Lepidium meyenii Walp,* which is a **Cruciferae** grow wild or cultivated in Andean Cordilleras of South America. The part to be used in the extraction can be any part such as a leaf, a flower, a fruit, a seed, a stem including rhizome and bulb. The bulb part is most preferably used. These ingredients can be crushed or cut into small pieces or powder for extraction. And these ingredients can be dried, if needed. Further, the Maca, which is previously extracted with water, can also be used.

Although, many extraction solutions to be used for obtaining the Maca extract can be listed, water-ethanol mixed aqueous solution (aqueous solution containing ethanol) is preferable from safety point of view, considering the fact that the extract would be comprised in food and beverage, and would be orally taken.

The inventors of the present invention had found that the potency of anti-allergic effect of the obtained extract differs greatly depending on the content ratio of the ethanol in the aqueous solution containing ethanol used as extraction solvent. In this case, it is important to maintain the mixture ratio of ethanol to water within the mixture ratio enables to obtain the extract having the high potency of anti-allergic effects. In concrete terms, the ratio is preferably from 20 to 100% by volume, and more preferably, from 45 to 100% by volume.

It is also important to maintain the temperature during the extraction within the condition enables to obtain the extract having the high potency of anti-allergic effects. It is preferable to maintain the temperature lower than boiling point of the solvent in the view of the operation. In concrete terms, the temperature of the solvent during the extraction is preferably from 20 to 75°C, and more preferably, from 40 to 60°C.

The mixing ratio of Maca and the solvent is not particularly limited, but it is preferred to use from 0.3 to 5,000 by weight% of solvent against 1 by weight% of Maca. Using from 5 to 100 by weight% of the solvent against 1 by weight% of Maca is more preferable in the view of extraction operation and extraction efficiency.

Extracting time is not limited. Although, there were examples of taking a few days to a few weeks to extract in the past, it takes only from 1 minute to 72 hours, or more specifically, from 10 minutes to 5 hours for the extraction by using extraction solvent of specific ethanol content ratio and maintaining within the specific extraction temperature based on the present invention.

By the way, the extract obtained by using water as the extraction solvent (either at room temperature or at high temperature) does not possess distinct anti-allergic effects. Therefore, it is preferable to remove impurities having no anti-allergic effect by extraction using water, before extracting operation using aqueous solution containing ethanol.

In the present invention, the Maca extract thus obtained after the completion of extracting operation can be used. Further, the concentrating extract by removing the solvent of the extract, or the dry powder by lyophilizing or air-drying of the extract solution can also be used. From the standpoints of stability and handling merits, it is preferable to use in the concentrating extract or the dry powder forms. The Maca extract used in this invention is to mean these extract solution, the concentrating extract, and the dry powder.

The present invention provides a food and beverage product, a perfumery and a cosmetic, and a pharmaceutical preparation comprising the Maca extract thus obtained. The Maca itself has been consumed in Peru from ancient time and therefore, the extract used in this invention has no problem in view of safety. Accordingly, the compounding quantity of the Maca extract to the food and beverage product, the perfumery and the cosmetic and the pharmaceutical preparation can be determined by its effect, aroma and color of the product to which the extract is added to. Preferable compounding quantity of Maca extract, however, is to be within concentration range of from 0.01 to 99.9% in dry weight, and more preferably from 0.01 to 99.5%.

The food and beverage product to be provided by the present invention is such as a candy, a troche, a gum, yogurt, a ice cream, a pudding, a jelly, a sweet jelly of beans, a alcoholic beverage, a coffee beverage, a juice, a fruit juice, a carbonated beverage, a refreshing beverage, a milk, a whey beverage, a lactic acid bacteria beverage and so on.

To these food and beverage product can be added various kinds of additives if needed according to conventional technique. The additives to be added can be any additives conventionally used according to conventional technique. The examples are glucose, fructose, saccharose, maltose, sorbitol, stevioside, rubsoside, corn syrup, lactose, citric acid, tartaric acid, malic acid, succinic acid, lactic acid, L-ascorbic acid, dl-$\alpha$-tocopherol, sodium erythorbate, glycerin, propylene glycol, glycerine fatty acid ester, polyglycerine fatty acid ester, sucrose fatty acid ester, sorbitan fatty acid ester, propylene glycol fatty acid ester, gum arabic, carrageenan, casein, gelatin, pectin, agar, a class of Vitamin B, nicotinic acid amide, calcium pantothenate, a class of amino acid, a class of calcium salt, coloring matter, aroma chemical and preservant and so on.

The perfumery and the cosmetic products of the present invention are any products called a perfumery and a cosmetic. The Maca extract can be comprised in appropriate quantity to confect a perfumery and a cosmetic which is not to put into mouth such as a skin toning lotion, a facial cream, a skin milk, a foundation, a lip stick, a hair dressing, a hair tonic, a medicated hair tonic, a shampoo, a conditioner and a bath agent, and which is to put into month such as a tooth brush and a tooth paste, a mouth wash, a gargle and a mouth refresher.

These products can be obtained according to the conventional technique comprising the common ingredient of a perfumery and a cosmetic. The examples are fats and oils such as a vegetable oil, a wax group such as lanoline and yellow beeswax, a carbon hydride group, a fatty acid, a higher alcohol group, an ester group, various surfactants, a coloring matter, an aroma chemical, a vitamin group, plant & animal extract, a ultraviolet absorption agent, an antioxidation agent, a antiseptic and a bactericide and so on.

When preparing a pharmaceutical preparation of the present invention, various additives and further the Maca extract can be comprised if needed. The present invention provides, for example, an oral administration pharmaceutical preparation such as a tablet, a capsule, granules, syrup and a extract agent, or a non-oral administration pharmaceutical preparation such as an ointment, an ocular ointment, a lotion, a cream, a patch, a rectal suppository, an ophthalmic drop, nasal drops and an injectable solution.

These pharmaceutical preparations can be produced according to conventional method using various additives. The additive to be used is not limited and any one commonly used can be suitable, examples being, solid carrier such as a starch, a lactose, a saccharose, a mannitol, a carboxymethyl cellulose, a corn starch and inorganic salts, distilled water, physiological saline, glucose aqueous solution and alcohol such as ethanol, liquid carriers such as propylene glycol and polyethylene glycol, various kinds of animal fat, a white petrolatum, a paraffin and oily carriers such as wax group.

Since the Maca extract of the present invention possess anti-allergic effects while it is safe in oral administration, it can be used as a food and beverage product, an orally administered pharmaceutical preparation and a perfumery and a cosmetic to put into mouth.

The dose and administration quantity of the Maca extract to be used per day is not limited. For example, it can be from 0.01mg to 10g per day in dry weight. Nevertheless, if one would take it orally and expects distinct anti-allergic effects, from 1mg to 1,000mg per day in dry weight should be preferably taken.

When preparing the food and beverage product, a perfumery and a cosmetic or pharmaceutical preparation by using the Maca extract of the present invention, other ingredient of a perfumery and a cosmetic having anti-inflammatory and anti-allergic effects such as an agar extract, especially glycyrrhizinate, a diphenhydramine hydrochloride, a dl-a-tocophenol and derivative thereof, Vitamin $B_2$ and Vitamin $B_6$ can also be used.

The additives or compounding agents for a food and beverage product, a perfumery, a cosmetic or pharmaceutical preparation in this invention mean the mixture of additives and compounding agents commonly used for a food and beverage product, a perfumery, a cosmetic or pharmaceutical preparation such as aroma chemicals, coloring agents and antioxidants with the Maca extract. The mixture ratio can be adjusted according to the purpose. The additives or compounding agents for the use in a food and beverage product, a perfumery, a cosmetic or pharmaceutical preparation discussed herein can be various additives listed above.

[Test Procedure]

The anti-allergic effects of the Maca extract of the present invention can be evaluated by the suppressive effect on the isolation of histamine from mast cells.
The test procedure is further described in detail.

The suppressive effect on the isolation of histamine from mast cells

The test was conducted according to the method of Yuko HIRAI, et al. (Natural Medicines: Vol.37, p374-380, 1983). That is, Tyrode was interperitoneally injected to Wister rats bled to death and peritoneal cavity cells were taken. Therefrom, mast cells were isolated by the multilevel centrifugal method using Bovine Serum Albumin (BSA)/physiological saline (relative gravity: 1.068). The obtained cells were suspended in 1% BSA containing Tyrode solution, and cell-floating solution was prepared. Then, 10μL of the cell-floating solution was added to 10μL of test solution and left for 10 minutes at 37°C, consequently, 20μL of compound 48/80 (5μL of/mL) as a degranulation inducing agent was added and reacted for 10 minutes at 37°C. The resultant was then ice cooled and amount of histamine in the supernatant after centrifugation (150g x 5 minutes) was measured by high performance liquid chromatography with fluorescence detector attachment.
The suppressive activity on the isolation of histamine was derived from below formula.

$$\text{The suppressive ratio on the isolation of histamine (\%)}$$
$$= 100 \times [1-(SR-C) / (R-C)]$$

Wherein:

C: amount of histamine isolated from the control cells
R: amount of histamine isolated from the cells when inducing agent is added
SR: amount of histamine isolated from the cell when inducing agent is added while the test portion is present.

Examples

The present invention will now be described in further detail with reference to Examples.

Example 1: Production of various kinds of the Maca extract

At first, the effect extraction conditions of the Maca such as extraction temperature and ethanol concentration have on anti-allergic effects of the Maca extract was studied.
10g of Maca was placed in 300mL conical flask, added 100mL of aqueous solutions with various ethanol concentration shown in Table 1, and then extracted for 3 hours at temperature shown in Table 1 to obtain Maca extract.
The Maca extracts of Test 10 and 11 were obtained from the residue of watery extract of Maca at 50°C for 1 hour, previously.
The resultant extract solution was filtrated and the filtrate was lyophilized to give the solutions of the present invention 1 to 11. The solution obtained was each measured to 0.25mg/mL, and the above explained suppressive effect on the isolation of histamine from mast cell was evaluated.
On the other hand, the above explained suppressive effect on the isolation of histamine from mast cell was evaluated for control solutions 1 to 3 which were obtained in the same procedures as for the solutions of the present invention except for extraction condition such as extraction temperature and ethanol concentration.
The result is also shown in Table 1.

Table 1:

| | Extraction Temperature (°C) | Ethanol concentration of extraction solvent (volume%) | Suppressive activity on isolation of histamine (%) |
|---|---|---|---|
| Control 1 | 50 | 0 | 31 |
| Control 2 | 90 | 0 | 0 |
| Control 3 | 60 | 16 | 7 |
| Test 1 | 60 | 30 | 18 |
| Test 2 | 60 | 45 | 70 |
| Test 3 | 60 | 60 | 76 |
| Test 4 | 60 | 95 | 83 |
| Test 5 | 60 | 100 | 78 |
| Test 6 | 40 | 60 | 56 |
| Test 7 | 75 | 20 | 29 |
| Test 8 | 25 | 100 | 31 |
| Test 9 | 20 | 60 | 16 |
| Test 10 | 20 | 99 | 20 |
| Test 11 | 60 | 99 | 81 |

As can be seen from the results, the solutions of the present invention 1 to 9 (Test 1 to 9), which were extracted with the extracting temperature being from 20 to 75°C and the ethanol concentration of extracting solvent being from 20% to 100%, show higher suppressive activities on the isolation of the histamine in comparison with the control solutions 1 to 3. Moreover, the solutions of the present invention 10 and 11, which are previously extracted by water, show the same high suppressive active. Especially, the solutions of the present invention 2 to 5 (Test 2 to 5), which were extracted with the t extracting temperature being 60°C and the specific ethanol concentration of extracting solvent being from 45% to 100%, and the solution of the present invention 6 (Test 6), which was extracted with the extracting temperature being 40°C and the specific ethanol concentration, of extracting solvent being 60%, show from 56% to 83% of the suppressive activity on the isolation of histamine and thus, the high potency of anti-allergic effect.

These results show very high potency of the suppressive effect on isolation of histamine compared to the suppressive effect on isolation of histamine (50%) of DSCG (0.1mg/mL) examined in the same line of test procedures.

From the above results, it was found that the Maca extract with the high potency of anti-allergic activities can be obtained by maintaining the temperature of the extraction solution and the ethanol concentration relatively high.

The Maca extract obtained is suitable to use in a food and beverage product, an oral administration pharmaceutical and a perfumery and a cosmetic to be placed in mouth, because it possesses the very high potency of anti-allergic effects and it uses ethanol aqueous solution which is safe.

Production Example 1: Pharmaceutical preparation comprising Maca extract

Tablet:

As shown below, a pharmaceutical preparation (tablet) comprising the Maca extract was prepared.

3kg of small pieces or powder of the Maca was placed in stainless container, to that 30L of aqueous solution having ethanol concentration of 99 by volume% was added, and mixed for 3 hors at 60°C. The resultant solution was filtrated and the solvent was removed from the extract to give 180g of the Maca extract of the present invention.

66.7g of the resultant Maca extract was then mixed with 232.0g of lactose and 1.3g of magnesium stearate and the mixture was compressed into tablets by a single punch tableting machine to give tablets with 10mm in diameter and 300mg in weight.

Granule:

0.5g of magnesium stearate was added to 99.5g of the Maca extract obtained by the above procedure, then compressed, crushed and sieved to give 20 to 50 mesh granules.

Production Example 2: Food and beverage products comprising the Maca extract

As shown below, food and beverage products comprising the Maca extract were prepared.

Candy:

| Composition | Weight% |
|---|---|
| Powdered sorbitol | 99.7 |
| Aroma chemical | 0.2 |
| Maca extract | 0.05 |
| Sorbitol seed | 0.05 |
| Total | 100.00 |

Candy:

| Composition | Weight% |
|---|---|
| Sugar | 47.0 |
| Starch syrup | 49.76 |
| Aroma chemical | 1.0 |
| Water | 2.0 |
| Maca extract | 0.24 |
| Total | 100.00 |

Troche:

| Composition | Weight% |
|---|---|
| Gum arabic | 6.0 |
| Glucose | 73.0 |
| Maca extract | 0.05 |
| Dibasic potassium phosphate | 0.2 |
| Primary potassium phosphate | 0.1 |
| Lactose | 17.0 |
| Aroma chemical | 0.1 |
| Magnesium stearate | 3.55 |
| Total | 100.00 |

Gum:

| Composition | Weight% |
| --- | --- |
| Gum base | 20.0 |
| Calcium carbonate | 2.0 |
| Stevioside | 0.1 |
| Maca extract | 0.05 |
| Lactose | 76.85 |
| Aroma chemical | 1.0 |
| Total | 100.00 |

Caramel:

| Composition | Weight% |
| --- | --- |
| Granulated sugar | 32.0 |
| Starch syrup | 20.0 |
| Powdered milk | 40.0 |
| Hardened oil | 4.0 |
| Salt | 0.6 |
| Aroma chemical | 0.02 |
| Water | 3.22 |
| Maca extract | 0.16 |
| Total | 100.00 |

Jelly (Coffee jelly):

| Composition | Weight% |
| --- | --- |
| Granulated sugar | 15.0 |
| Gelatin | 1.0 |
| Coffee extract | 5.0 |
| Water | 78.93 |
| Maca extact | 0.07 |
| Total | 100.00 |

Ice cream:

| Composition | Weight% |
|---|---|
| Fresh cream (45% fat) | 33.8 |
| Skim milk | 11.0 |
| Granulated sugar | 14.8 |
| Sweetened egg yolk | 0.3 |
| Vanilla essence | 0.1 |
| Water | 39.93 |
| Maca extract | 0.07 |
| Total | 100.00 |

Custard pudding:

| Composition | Weight% |
|---|---|
| Milk | 47.51 |
| Egg | 31.9 |
| White superior soft sugar | 17.1 |
| Water | 3.4 |
| Maca extract | 0.09 |
| Total | 100.00 |

Sweet jelly of beans:

| Composition | Weight% |
|---|---|
| Red bean jam | 24.8 |
| Powdered agar | 0.3 |
| Salt | 0.1 |
| White superior soft sugar | 24.9 |
| Maca extract | 0.1 |
| Water | 49.8 |
| Total | 100.00 |

Juice:

| Composition | Weight% |
|---|---|
| Frozen concentrated juice of Unshu orange | 5.0 |
| Fructose/glucose liquid sugar | 11.0 |
| Citric acid | 0.2 |
| L-ascorbic acid | 0.02 |

(continued)

| Composition | Weight% |
|---|---|
| Maca extract | 0.05 |
| Aroma chemical | 0.2 |
| Coloring agent | 0.1 |
| Water | 83.43 |
| Total | 100.00 |

Carbonated beverage:

| Composition | Weight% |
|---|---|
| Granulated sugar | 8.0 |
| Concentrated juice of lemon | 1.0 |
| L-ascorbic acid | 0.10 |
| Citric acid | 0.09 |
| Sodium citrate | 0.05 |
| Coloring agent | 0.05 |
| Aroma chemical | 0.15 |
| Carbonated water | 90.55 |
| Maca extract | 0.01 |
| Total | 100.00 |

Lactic acid bacteria beverage:

| Composition | Weight% |
|---|---|
| Cultured milk 21% lactic solid content | 14.76 |
| Fructose - glucose liquid sugar | 13.31 |
| Pectin | 0.5 |
| Citric acid | 0.08 |
| Aroma chemical | 0.15 |
| Water | 71.14 |
| Maca extract | 0.06 |
| Total | 100.00 |

Coffee beverage:

| Composition | Weight% |
|---|---|
| Granulated sugar | 8.0 |

(continued)

| Composition | Weight% |
|---|---|
| Skim milk | 5.0 |
| Caramel | 0.2 |
| Coffee extract | 2.0 |
| Aroma chemical | 0.1 |
| Polyglycerin fatty acid ester | 0.05 |
| Salt | 0.05 |
| Water | 84.56 |
| Maca extract | 0.04 |
| Total | 100.00 |

Alcoholic beverage:

| Composition | Weight% |
|---|---|
| 50 by valume% of ethanol | 32.0 |
| Sugar | 8.4 |
| Juice | 2.4 |
| Maca extract | 0.2 |
| Purified water | 57.0 |
| Total | 100.00 |

Production Example 3: Perfumery and cosmetic comprising Maca extract

As shown below, perfumery and cosmetic comprising Maca extract is prepared.

Toothpaste:

| Composition | Weight% |
|---|---|
| Dibasic calcium phosphate | 42.0 |
| Glycerin | 18.0 |
| Carrageenan | 0.9 |
| Sodium lauryl sulfate | 1.2 |
| Saccharin sodium | 0.09 |
| P-hydroxybenzoate butyl | 0.005 |
| Maca extract | 0.05 |
| Aroma chemical | 1.0 |
| Water | 36.755 |
| Total | 100.00 |

Mouthwash:

| Composition | Weight% |
|---|---|
| Sodium lauryl sulfate | 0.8 |
| Glycerin | 7.0 |
| Sorbitol | 5.0 |
| Ethanol | 15.0 |
| Maca extract | 0.05 |
| 1-menthole | 0.05 |
| Aroma chemical | 0.04 |
| Saccharin sodium | 0.1 |
| Water | 71.96 |
| Total | 100.00 |

Facial Cream (acidulous):

| Composition | Weight% |
|---|---|
| Glycerin | 5.0 |
| Propylene glycol | 4.0 |
| Hyaluronate sodium | 0.1 |
| Maca extract | 0.05 |
| Polyoxyethylene sorbitan ester monolaurate (20EO) | 1.5 |
| Polyoxyethylene lauryl Ether (20EO) | 0.5 |
| Ethanol | 10.0 |
| Aroma chemical | 0.1 |
| Coloring agent | *ad lib.* |
| Antiseptic | *ad lib.* |
| Ultraviolet absorption agent | *ad lib.* |
| Purified water | 78.75 |
| Total | 100.00 |

Emollient cream:

| Composition | Weight% |
|---|---|
| Yellow beeswax | 2.0 |
| Stearyl alcohol | 5.0 |
| Stearic acid | 8.0 |
| Squalane | 10.0 |

(continued)

| Composition | Weight% |
|---|---|
| Self-emulsifying propylene glycol monostearate | 3.0 |
| Polyoxyethlene cetyl ether (20EO) | 1.0 |
| Aroma chemical | 0.5 |
| Antioxidant | *ad lib.* |
| Antiseptic | *ad lib.* |
| Propylene glycol | 4.8 |
| Glycerin | 3.0 |
| Hyaluronate sodium | 0.1 |
| Maca extract | 0.1 |
| Triethanolamine | 1.0 |
| Purified water | 61.5 |
| Total | 100.00 |

Emollient lotion:

| Composition | Weight% |
|---|---|
| Stearate | 2.0 |
| Cetyl alcohol | 1.5 |
| Vaseline | 3.0 |
| Lanoline alcohol | 2.0 |
| Liquid parrafin | 10.0 |
| Polyoxyethylene mono esther oleate (10EO) | 2.0 |
| Aroma chemical | 0.5 |
| Antioxidant | *ad lib.* |
| Antiseptic | *ad lib.* |
| Propylene glycol | 4.8 |
| Glycerin | 3.0 |
| Hyaluronate sodium | 0.1 |
| Maca extract | 0.1 |
| Triethanolamine | 1.0 |
| Purified water | 70.0 |
| Total | 100.00 |

Liquid foundation:

| Composition | Weight% |
|---|---|
| Stearate | 2.4 |
| Monostearate propylene glycol | 2.0 |
| Cetostearyl alcohol | 0.2 |
| Liquid lanoline | 2.0 |
| Liquid paraffin | 3.0 |
| Isopropyl myristate | 8.5 |
| P-hydroxybenzoate propyl | *ad lib.* |
| Purified water | 64.1 |
| Carboxymethyl cellulose | 0.2 |
| Bentonite | 0.5 |
| Propylene glycol | 3.8 |
| Hyaluronate sodium | 0.1 |
| Maca extract | 0.1 |
| Triethanolamine | 1.1 |
| P-hydroxybenzoate methyl | *ad lib.* |
| Titanium oxide | 8.0 |
| Talc | 4.0 |
| Coloring agent | *ad lib.* |
| Aroma chemical | *ad lib.* |
| Total | 100.00 |

Hair-growth tonic:

| Composition | Weight% |
|---|---|
| Ethanol | 70.0 |
| Sialid extract | 0.2 |
| Vitamin E | 0.2 |
| 1-menthol | 0.4 |
| Glycyrrhizin | 0.1 |
| Maca extract | 0.1 |
| 2-hydroxybenzoic acid | 0.5 |
| Glycerin | 0.5 |
| Aroma chemical | *ad lib.* |
| Purified water | 28.0 |
| Total | 100.00 |

Shampoo:

14

| Composition | Weight% |
|---|---|
| Alkylether sodium sulfate | 16.0 |
| Diethanoramide laurate | 4.0 |
| Propylene glycol | 1.9 |
| Maca extract | 0.1 |
| Antiseptic | *ad lib.* |
| Coloring agent | *ad lib.* |
| Aroma chemical | *ad lib.* |
| Purified water | 78.0 |
| Total | 100.00 |

Conditioner:

| Composition | Weight% |
|---|---|
| Stearyl dimethyl chloride | 1.4 |
| Bezyl ammonium stearyl-alcohol | 0.6 |
| Glyceric monostearate | 1.5 |
| Salt | 0.1 |
| Maca extract | 0.1 |
| Purified water | 96.3 |
| Total | 100.00 |

INDUSTRIAL APPLICABILITY

As set forth, the present invention provides the Maca extract possessing the high potency of anti-allergic effects produced by the extracting procedure of the present invention.

Since the obtained Maca extract possesses strong anti-allergic effects, a food and beverage product, a perfumery and a cosmetic and a pharmaceutical preparation containing thereof suppress allergic symptoms of everyday life very effectively, and thus this invention contributes enormously to the industry.

**Claims**

1. Extraction method of Maca extract, wherein a temperature of extracting solution is from 20°C to 75°C when Maca extract is extracted by adding aqueous solution containing ethanol to small pieces or powder of Maca.

2. The extraction method according to claim 1, wherein concentration of the ethanol in the aqueous solution containing ethanol is from 20 to 100 by volume%.

3. The extraction method according to claim 1, wherein from 0.3 to 500 by weight% of the aqueous solution containing ethanol is used against 1 by weight% of small pieces or powder or small piece of Maca.

4. The extraction method according to any one of claims 1 to 3, wherein the small pieces or powder of Maca is a residue of watery extraction.

5. A Maca extract obtained by the method according to any one of claims 1 to 4.

**6.** A food and beverage product, a perfumery and a cosmetic and a pharmaceutical preparation comprising the Maca extract of claim 5.

**7.** A food and beverage product, a perfumery and a cosmetic and a pharmaceutical preparation according to claim 6, wherein from 0.01 to 99.5% of Maca extract according to claim 5 in dry weight is comprised.

**8.** A food and beverage product, a perfumery and a cosmetic and a pharmaceutical preparation according to either claim 6 or 7, possessing an anti-allergic effect.

**9.** An additive or a compounding agent for a food and beverage product, a perfumery and a cosmetic, or a pharmaceutical preparation, wherein Maca extract according to claim 5 is comprised.

**10.** A food and beverage product, a perfumery and a cosmetic or a pharmaceutical preparation according to claim 8, being a candy, a troche, a gum, a granule or a tablet.

## INTERNATIONAL SEARCH REPORT

| | International application No. |
|---|---|
| | PCT/JP2005/001201 |

A.   CLASSIFICATION OF SUBJECT MATTER
   Int.Cl⁷  A61K7/00, 35/78, A23G3/00, A23L1/30, A61P37/08

According to International Patent Classification (IPC) or to both national classification and IPC

B.   FIELDS SEARCHED

Minimum documentation searched (classification system followed by classification symbols)
   Int.Cl⁷  A61K7/00, 35/78, A23G3/00, A23L1/30, A61P37/08

Documentation searched other than minimum documentation to the extent that such documents are included in the fields searched

Electronic data base consulted during the international search (name of data base and, where practicable, search terms used)
   CA(STN), BIOSIS(STN), MEDLINE(STN), EMBASE(STN), JICST(JOIS)

C.   DOCUMENTS CONSIDERED TO BE RELEVANT

| Category* | Citation of document, with indication, where appropriate, of the relevant passages | Relevant to claim No. |
|---|---|---|
| X | WO 00/51548 A2   (Pure World Botanical INC.),<br>08 September, 2000 (08.09.00),<br>p5 1.30-31, p10.1.7-9, p13; examples 1 to 3<br>& EP 1180006 A          & CA 2362858 A<br>& AU 3864900 A          & US 6267995 B1<br>& US 2002/42530 A1      & US 2002/61341 A1<br>& US 2003/68388 A1 | 1-10 |
| X | JP 2004-171 A  (Towa Shoji Kabushiki Kaisha),<br>08 January, 2004 (08.01.04),<br>Page 3; Par. No. [0020]<br>(Family: none) | 1-10 |
| X<br>Y | JP 2002-161043 A  (Kabushiki Kaisha TS Asu),<br>04 June, 2002 (04.06.02),<br>Page 3; preparation example 4; test 1<br>(Family: none) | 1-3,5-10<br>4 |

☒  Further documents are listed in the continuation of Box C.          ☐  See patent family annex.

| | |
|---|---|
| *          Special categories of cited documents:<br>"A"   document defining the general state of the art which is not considered<br>       to be of particular relevance<br>"E"   earlier application or patent but published on or after the international<br>       filing date<br>"L"   document which may throw doubts on priority claim(s) or which is<br>       cited to establish the publication date of another citation or other<br>       special reason (as specified)<br>"O"   document referring to an oral disclosure, use, exhibition or other means<br>"P"   document published prior to the international filing date but later than the<br>       priority date claimed | "T"   later document published after the international filing date or priority<br>       date and not in conflict with the application but cited to understand<br>       the principle or theory underlying the invention<br>"X"   document of particular relevance; the claimed invention cannot be<br>       considered novel or cannot be considered to involve an inventive<br>       step when the document is taken alone<br>"Y"   document of particular relevance; the claimed invention cannot be<br>       considered to involve an inventive step when the document is<br>       combined with one or more other such documents, such combination<br>       being obvious to a person skilled in the art<br>"&"   document member of the same patent family |

| Date of the actual completion of the international search<br>   17 February, 2005 (17.02.05) | Date of mailing of the international search report<br>   08 March, 2005 (08.03.05) |
|---|---|
| Name and mailing address of the ISA/<br>   Japanese Patent Office | Authorized officer |
| Facsimile No. | Telephone No. |

Form PCT/ISA/210 (second sheet) (January 2004)

## INTERNATIONAL SEARCH REPORT

| | International application No. |
|---|---|
| | PCT/JP2005/001201 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2001-39854 A  (Sanko Bussan Kabushiki Kaisha),<br>13 February, 2001 (13.02.01),<br>Pages 5 to 7; preparation examples 2 to 5;<br>experimental example 2<br>(Family: none) | 1-3,5-10<br>4 |
| X<br>Y | Ayanari SUZUKI et al., "Maka Chushutsubutsu no Rat Sokuseki Fushu ni Taisuru Koensho Sayo",<br>Medicine and Biology, 2002, Vol.145, No.6,<br>pages 81 to 86 | 1-3,5-10<br>4 |
| X<br>Y | JP 8-12565 A  (Shiseido Co., Ltd.),<br>16 January, 1996 (16.01.96),<br>Page 4; Par. No. [0027]<br>(Family: none) | 1-3,5-10<br>4 |
| P,X | Yudai MATSUMOTO et al., "Maka (Lepidium meyennii Walp) no Ko-Allergy Kassei",<br>Nippon Yakugakukai Nenkai Koen Yoshishu,<br>05 March, 2004 (05.03.04), Vol.124th,<br>No.2, page 113 | 1-10 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6192114 A

- US 6267995 B1

**Non-patent literature cited in the description**

- *Igaku & Seibutsugaku,* 10 December 2002, vol. 145 (6), 81-86

- **YUKO HIRAI et al.** *Natural Medicines,* 1983, vol. 37, 374-380